(19) Europäisches Patentamt / European Patent Office / Office européen des brevets

(11) **EP 1 829 982 B1**

(12) **EUROPEAN PATENT SPECIFICATION**

(45) Date of publication and mention
of the grant of the patent:
**30.01.2013 Bulletin 2013/05**

(51) Int Cl.:
*C22C 19/07* *(2006.01)*  *A61L 27/04* *(2006.01)*
*C21D 6/00* *(2006.01)*  *C22C 38/00* *(2006.01)*
*C22F 1/10* *(2006.01)*

(21) Application number: **05727390.6**

(22) Date of filing: **28.03.2005**

(86) International application number:
**PCT/JP2005/005784**

(87) International publication number:
**WO 2006/054368 (26.05.2006 Gazette 2006/21)**

(54) **BIO-Co-Cr-Mo ALLOY WITH ION ELUTION SUPPRESSED BY REGULATION OF TEXTURE, AND PROCESS FOR PRODUCING THE SAME**

BIO-CO-CR-MO-LEGIERUNG MIT DURCH TEXTURREGULIERUNG UNTERDRÜCKTER IONENELUTION UND HERSTELLUNGSVERFAHREN DAFÜR

ALLIAGE BIO-CO-CR-MO A ELLUTION IONIQUE SUPPRIMEE PAR REGULATION DE LA TEXTURE ET SON PROCEDE D'OBTENTION

(84) Designated Contracting States:
**CH DE FR LI**

(30) Priority: **19.11.2004 PCT/JP2004/017302**

(43) Date of publication of application:
**05.09.2007 Bulletin 2007/36**

(73) Proprietor: **Iwate University**
**Morioka-shi**
**Iwate 020-8550 (JP)**

(72) Inventors:
 • **CHIBA, Akihiko**
  **Morioka-shi Iwate 020-8550 (JP)**
 • **KUROSU, Shingo**
  **Morioka-shi Iwate 020-0115 (JP)**
 • **NOMURA, Naoyuki**
  **Morioka-shi Iwate 020-8550 (JP)**

(74) Representative: **WSL Patentanwälte**
**Partnerschaftsgesellschaft**
**Postfach 6145**
**65051 Wiesbaden (DE)**

(56) References cited:
 **WO-A-00/47140** **JP-A- 4 013 416**
 **JP-A- 56 069 343** **JP-A- 2001 512 529**

JP-A- 2002 235 153  JP-B2- 04 013 416
US-A- 2 246 288  US-A- 4 530 664

• **KULMBURG A ET AL: "The Microstructure of Co-Cr-Mo-(Nb) Dental Alloys" PRAKTISCHE METALLOGRAPHIE, HANSER, MUENCHEN, DE, vol. 38, no. 9, 1 January 2001 (2001-01-01), pages 514-531, XP002999572 ISSN: 0032-678X**
• **KUMAGAI K. ET AL.: 'H.C.P. Tanso Soshiki o Yusuru Seitaiyo Co-Ki Gokin no Mamo Tokusei' THE JAPAN INSTITUTE OF METALS KOEN GAIYO vol. 134TH, 30 March 2004, page 119, XP002999568**
• **KUROSU S. ET AL.: 'Yoto Tanzo ni yotte Sakusei sareta Seitaiyo Co Gokin no Giji Seitai Ekichu ni Okeru Fushoku Kyodo' THE JAPAN INSTITUTE OF METALS KOEN GAIYO vol. 135TH, 28 September 2004, page 463, XP002999569**
• **ABE Y. ET AL.: 'Yoto Tanzo ni yote sakusei shita Seitaiyo Co-CR-Mo Gokin no Soshiki to Kikaiteki Kyodo ni Oyobosu Netsushori no Eikyo' THE JAPAN INSTITUTE OF METALS KOEN GAIYO vol. 135TH, 28 September 2004, page 463, XP002999570**
• **SUZUKI K. ET AL.: 'Giji Taieki Chu ni Okeru Stainless-ko Seitai Shigeki Denkyoku no Hiro Tokusei' THE JAPAN INSTITUTE OF METALS KOEN GAIYO vol. 131ST, 02 November 2002, page 464, XP002999571**
• **KULMBURG A. ET AL.: 'The Microstructure of Co-Cr-Mo-(Nb) Dental Alloys' PRAKT. METALLOGR. vol. 38, no. 9, 2001, pages 514 - 531, XP002999572**

## Description

TECHNICAL FIELD

The present invention relates to a method for neutralizing allergy toxicity and other bio-toxicity due to Ni trace impurities in a bio-Co-Cr-Mo alloy or nickel-free stainless steel alloy, to a Co-Cr-Mo alloy or Ni-free stainless steel alloy in which bio-toxicity is neutralized, and to a bio material and artificial prosthesis material manufactured from the alloy.
The present invention provides a technique for suppressing allergic reaction through the use of a technique for controlling the structure of a Co-Cr-Mo alloy, whereby an ε phase, which is a crystal structure having a low ion elution rate, is actively utilized to reduce the rate of ion elution from the surface of a Co-Cr-Mo alloy implanted in a body.

BACKGROUND ART

Co-Cr-Mo alloy has excellent reliability with regard to corrosion resistance, wear resistance, and workability, and is therefore used in artificial hip joint and other regions that have sliding surfaces; artificial aggregate prosthetic materials; and surgical implants and various other medical devices. Plastic forming of a Co-base alloy is difficult, and Ni has therefore been added to improve working properties. However, allergies, carcinogenicity, and other bio-toxicity due to Ni have recently been reported. Attempts are therefore being made to develop materials that have no added Ni.

However, Ni is unavoidably included in the starting material even when not added on purpose, and the bio-toxicity of even such trace amounts of Ni is a cause for concern. For instance WO 00/47 140 discloses a Co-Cr-Mo alloy with up to 1.0 wt:/. Ni as impurity and which also contains titanium in a small amount. The problem of the unavoidable presence of Ni in the starting material can be overcome in theory by increasing the purity of the starting material, but increasing the purity in this manner leads to increased cost of the starting material, and is therefore impractical and problematic. In the same manner, Ni-free stainless steel alloy is also used in bio-materials and medical materials due to the excellent characteristics of Ni-free stainless steel alloy, and is viewed hopefully. However, Ni is also unavoidably present in the starting material, and although the alloy is referred to as "Ni-free," bio-toxicity actually occurs due to trace amounts of Ni that cannot be prevented from occurring.

Co-Cr-Mo alloy is highly resistant to corrosion and wear, and is therefore used in various medical devices. The use of Co-Cr-Mo alloy is particularly common in artificial joint materials. A recently identified problem is that of allergies caused by elution into the body of Ni as a trace impurity that unavoidably exists on the order of several hundred parts per million, or of ions of Co, which is a main constituent element of the Co-Cr-Mo alloy. There is a need to develop a method for suppressing such ion elution from the surface of a bio-Co-Cr-Mo alloy, and a method must be developed to prevent allergic reactions from occurring as a result of ion elution.

DISCLOSURE OF THE INVENTION

[Problems To Be Solved By The Invention]

The inventors therefore performed a wide range of concentrated investigations aimed at developing a technique for suppressing toxicity caused by Ni in a bio-Co-Cr-Mo alloy or the like. As a result, the inventors developed the present invention upon discovering that toxicity caused by Ni can be suppressed without compromising the excellent characteristics of an alloy by adding an element that forms a compound with Ni and has minimal bio-toxicity to a bio-Co-Cr-Mo alloy. The inventors also developed the present invention with the awareness that the detoxification technique can also be applied to a Ni-free stainless steel alloy.

The inventors performed further investigation aimed at controlling the elution of ions that cause problems in the body, and performed detailed investigation of a Co-Cr-Mo alloy with regard to structural control, i.e., the ion elution behavior of γ phases and c phases that occur in the Co-Cr-Mo alloy. As a result, the inventors developed the present invention upon discovering that the ion elution rate of ε phases is significantly lower than that of the γ phases, and thereby recognizing that the elution of ions can be controlled to suppress allergic reactions by controlling the structure of the alloy.

The present invention is given in the claims.

The present invention provides a method for neutralizing bio-toxicity due to Ni trace impurity in a bio-Co-Cr-Mo alloy or a Ni-free stainless steel alloy, wherein the method for neutralizing nickel toxicity of a bio-Co-Cr-Mo alloy or Ni-free stainless steel alloy is characterized in comprising adding Ti or Zr to the alloy. In particular, the present invention provides a method for neutralizing bio-toxicity due to Ni trace impurity in a bio-Co-Cr-Mo alloy or a Ni-free stainless steel alloy, wherein the method for neutralizing nickel toxicity in a bio-Co-Cr-Mo alloy or a Ni-free stainless steel alloy is characterized in that an element selected from the group that consists Ti or Zr. The additive element is selected from the group that includes zirconium and titanium. The additive element is more preferably zirconium. In particular, the present invention provides a technique for neutralizing nickel toxicity that is applied to an alloy in which a nickel content in the alloy composition is (1) about 1.0 wt% or less, (2) about 0.5 wt% or less, (3) about 0.002 wt% or less, (4) at least on the order of 100 ppm or less, or (5) on the order of several hundred parts per million or less; and the alloy composition is an alloy in which Ni is unavoidably present.

The present invention provides a bio-Co-Cr-Mo alloy or Ni-free stainless steel alloy in which nickel toxicity is neutralized, characterized in comprising a bio-Co-Cr-Mo alloy or Ni-free stainless steel alloy in which Zr or Ti is added to an alloy. composition in order to neutralize bio-toxicity due to Ni trace impurity. In one embodiment, the alloy of the present invention comprises an alloy having a nickel content of (1) about 1.0 wt% or less, (2) about 0.5 wt% or less, (3) about 0.002 wt% or less, (4) at least on the order of 100 ppm or less, or (5) on the order of several hundred parts per million or less; wherein Ni is unavoidably present in the alloy. The present invention furthermore provides a medical device manufactured from the bio-Co-Cr-Mo alloy or Ni-free stainless steel alloy in which nickel toxicity is neutralized. The present invention also provides a medical device manufactured by subjecting the bio-Co-Cr-Mo alloy or Ni-free stainless steel alloy in which nickel toxicity is neutralized to a process selected from the group that includes quenching, metal gas atomization, mechanical alloying, liquid quenching, hot extrusion, hot rolling, hot drawing, and forging.

The present invention also has such aspects as those described below.

The present invention provides a method for suppressing ion elution in a bio-Co-Cr-Mo alloy, wherein the method for suppressing ion elution from a bio-Co-Cr-Mo alloy is characterized in comprising adjusting an alloy structure in controlled fashion to cause enrichment with an ε HCP phase structure. In a preferred embodiment, adjusting an alloy structure in a bio-Co-Cr-Mo alloy in controlled fashion can be achieved by (1) adding Zr or Ti to the alloy composition and/or (2) performing appropriate heat treatment. The additive element is selected from the group that includes Ti and Zr. The additive element is selected from the group that includes zirconium and titanium. The additive element is preferably zirconium. The nickel content in the alloy composition may, of course, be such as described above. Control of the Co-Cr-Mo alloy structure may include performing heat treatment at a temperature of 600°C to 1250°C after alloy melting. The control of the Co-Cr-Mo alloy structure may also include (i) melting an alloy composition or heat treating an alloy composition at a temperature of 1000°C or higher, and then rapidly cooling the alloy composition; or (ii) heat treating an alloy composition for a long period of time at a temperature of approximately 1000°C or higher and in a temperature range of at least 550 to 650°C.

The present invention provides a bio-Co-Cr-Mo alloy characterized in that an alloy structure in the bio-Co-Cr-Mo alloy is enriched with an ε HCP phase structure, and ion elution from the alloy is suppressed or reduced. For example, the alloy is one in which Zr or Ti is added to a bio-Co-Cr-Mo alloy composition. The alloy may be one in which heat treatment at a temperature of 600°C to 1250°C is performed after alloy melting. The alloy may also be one in which (i) an alloy composition is melted or heat treated at a temperature of 1000°C or higher, and then rapidly cooled; or (ii) an alloy composition is heat treated for a long period of time at a temperature of approximately 1000°C or higher and in a temperature range of at least 550 to 650°C. The present invention provides a medical device manufactured from a bio-Co-Cr-Mo alloy that is enriched with an ε HCP phase structure, and in which ion elution from the alloy is suppressed or reduced. As described above, this device may be manufactured by subjecting the bio-Co-Cr-Mo alloy to a process selected from the group that includes quenching, metal gas atomization, mechanical alloying, liquid quenching, hot extrusion, hot rolling, hot drawing, and forging.

[Effect of the Invention]

Ni can be fixed, and elution of Ni ions can be suppressed by adding an element having minimal bio-toxicity, e.g., Ti or Zr, as a fourth element to a Co-Cr-Mo alloy or a Ni-free stainless steel alloy. The toxicity of Ni is thereby essentially neutralized. Ni impurities (on the order of 100 ppm) introduced from the starting material are present even when Ni is not intentionally added, and the present invention also compensates for such Ni. The present invention also provides a technique for suppressing allergic reaction through the use of a technique for controlling the structure of a Co-Cr-Mo alloy, whereby an ε phase, which is a crystal structure having a low ion elution rate, is actively utilized to reduce the rate of ion elution from the surface of a Co-Cr-Mo alloy implanted in a body. The present invention can thus be applied as a bio-material having minimal bio-toxicity, i.e., increased safety, in artificial hip joints, stents, and various other medical devices.

Other objects, characteristics, advantages, and aspects of the present invention will be apparent to one skilled in the art from the description given below. However, it should be understood that the following description and the description of the present specification, which includes specific examples and the like, merely show preferred modes of the present invention and are given only by way of explanation. It will be clearly apparent to one skilled in the art from the information given in the following description and other portions of the present specification that various changes and/or improvements (or modifications) are possible within the scope of the present invention as disclosed in the claims. All patent references and other references cited in the present specification are cited for descriptive purposes.

BRIEF DESCRIPTION OF THE DRAWINGS

FIG. 1 shows the Co metal elution results for alloy test samples (Example 1) in metal elution testing using 1% lactic acid;
FIG. 2 shows the Cr metal elution results for alloy test samples (Example 1) in metal elution testing using 1% lactic acid;

FIG. 3 shows the Mo metal elution results for alloy test samples (Example 1) in metal elution testing using 1% lactic acid;

FIG. 4 shows the Ni metal elution results for alloy test samples (Example 1) in metal elution testing using 1% lactic acid;

FIG. 5 shows the metal elution results of each additive element for alloy test samples (Example 1) in metal elution testing using 1% lactic acid;

FIG. 6 shows the nominal stress/nominal strain curves for Co-29Cr-6Mo-1Ni alloy and Co-29Cr-6Mo-1Ni-.05Zr alloy;

FIG. 7 shows the Co metal elution results for alloy test samples (Example 3) in metal elution testing using 1% lactic acid;

FIG. 8 shows the Cr metal elution results for alloy test samples (Example 3) in metal elution testing using 1% lactic acid;

FIG. 9 shows the Mo metal elution results for alloy test samples (Example 3) in metal elution testing using 1% lactic acid;

FIG. 10 shows the Ni metal elution results for alloy test samples (Example 3) in metal elution testing using 1% lactic acid;

FIG. 11 shows the effects of the additive element (per atomic percent) on the HCP-to-FCC phase transformation temperature of Co, wherein the vertical axis indicates the solution limit of the additive element, and the horizontal axis indicates the change in the HCP-to-FCC phase transformation temperature due to addition of 1.0% of the additive element; reference: C. T. Sims, N. S. Stoloff & W. C. Hagel, SUPERALLOYS II, Wiley-Interscience (1987);

FIG. 12 shows optical micrographs of the structure of (a) an alloy having the composition Co-29 wt% Cr-6 wt% Mo-1 wt% Ni; (b) an alloy having the composition Co-29 wt% Cr-6 wt% Mo-1 wt% Ni-0.3 wt% Nb; and (c) an alloy having the composition Co-29 wt% Cr-6 wt% Mo-1 wt% Ni-0.1 wt% Zr;

FIG. 13 shows the metal elution results of each additive element for alloy test samples (Example 4) in metal elution testing using 1% lactic acid;

FIG. 14 shows optical micrographs of the structure of (a) an alloy having the composition Co-29 wt% Cr-6 wt% Mo-1 wt% Ni; (b) an alloy having the composition Co-29 wt% Cr-6 wt% Mo-1 wt% Ni-0.05 wt% Zr; (c) an alloy having the composition Co-29 wt% Cr-6 wt% Mo-1 wt% Ni-0.1 wt% Zr; and (d) an alloy having the composition Co-29 wt% Cr-6 wt% Mo-1 wt% Ni-0.3 wt% Zr; and

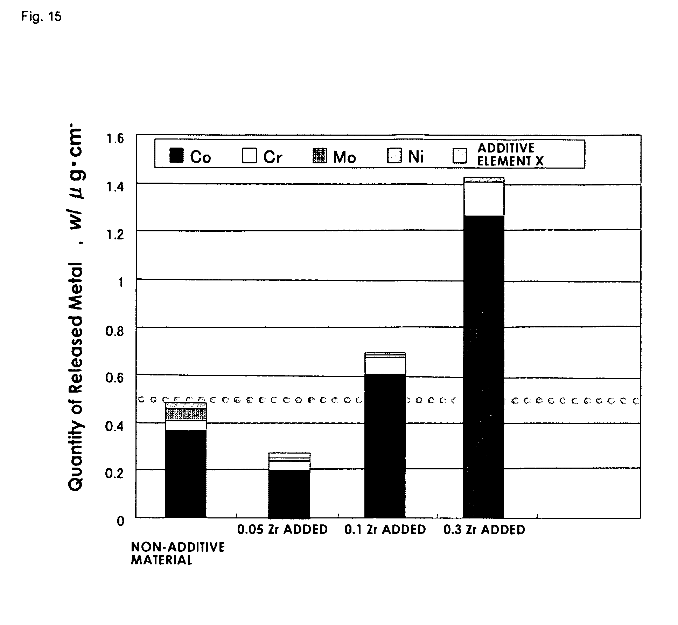

FIG. 15 shows the metal elution results of each additive element for alloy test samples (Example 5) in metal elution testing using 1% lactic acid.

## BEST MODE FOR CARRYING OUT THE INVENTION

In the present invention, the "bio-Co-Cr-Mo alloy" is a cobalt-based (Co-based) alloy that includes chromium (Cr) and molybdenum (Mo) in substantial ratios, and includes alloys that are known in the field as "super alloys." The term "super alloy" is a technical term that refers generically to alloys that have extremely high strength as well as excellent mechanical characteristics and corrosion resistance; and typical super alloys are recognized as having a stable micro-structure. A bio-Co-Cr-Mo alloy has excellent biological compatibility, high yield strength, excellent hardness, and other properties. ASTM (American Society for Testing and Material) specifications, e.g., ASTM F1537 94, ASTM F799, ASTM F75, and others; and ISO (International Organization for Standardization) specifications, e.g., ISO 5832-12 and others, can be cited as specifications of the Co-Cr-Mo alloy.

The alloy composition (weight% (wt%)) specified by ASTM F 1537 94 has such a composition as the following:

Mo: 5.0 to 7.0 wt%, Cr: 26.0 to 30.0 wt%, C: $\leq$ 0.35 wt%,
Ni: $\leq$ 1.0 wt%, Fe: $\leq$ 0.75 wt%, Mn: $\leq$ 1.0 wt%,
Si: $\leq$ 1.0 wt%, $N_2$: $\leq$ 0.25 wt%, Co: balance.

In this composition, due to the fact that Ni is unavoidably present in the starting material, a ratio of at least 0.2 to 1.0 wt% of Ni is usually included, and the "Co: balance" is the amount of Co minus trace amounts of incidental impurities.

The alloy Vitallium (proprietary name) is known as a Co-Cr-Mo alloy that is an orthopedic surgical product, and the general composition thereof is as follows:

Mo: approximately 5.50 wt%, Cr: approximately 28.00 wt%,
C: approximately 0.25 wt%, Mn: approximately 0.70 wt%,
Si: approximately 0.75 wt%, Co: balance.

In this composition, due to the fact that Ni is unavoidably present in the starting material, a ratio of at least 0.002 to 2.5 wt% of Ni is usually included, and the "Co: balance" is the amount of Co minus trace amounts of incidental impurities.

Numerous Co-Cr-Mo alloys have been reported, and examples of the Co-Cr-Mo alloy include products, modifications of products, or derivatives of products disclosed in Japanese Patent Application Laid-Open No. 2002-363675 (JP A 2002-363675), International Publication WO 97/00978 pamphlet (WO A 97/00978), specification of US Patent No. 5,462,575 (US A 5462575), specification of US Patent No. 4,668,290 (US A 4668290), and other publications. For example, as described in Japanese Patent Application Laid-Open No. 2002-363675, alloys are included in which the amount of Mo is increased to about $\leq$ 12.0 wt%, or to about 10 wt%.

In one specific example, the Co-Cr-Mo alloy may have the composition described below. Mo: approximately 5.0 to 6.0 wt%, Cr: approximately 26.0 to 28.0 wt%, C: ≤ approximately 0.07 wt%, Ni: ≤ approximately 1.0 wt%, Fe: ≤ approximately 0.75 wt%, Mn: ≤ approximately 1.0 wt%, Si: ≤ approximately 1.0 wt%, N2: ≤ approximately 0.25 wt%, Co: balance (in this composition, due to the fact that Ni is unavoidably present in the starting material, a ratio of at least about 0.002 wt% and at least an amount greater than an amount on the order to 50 ppm is usually included, and the "Co: balance" is the amount of Co minus trace amounts of incidental impurities).

In another specific example, the Co-Cr-Mo alloy may have the composition described below. Mo: approximately 6.0 to 12.0 wt%, Cr: approximately 26.0 to 30.0 wt%, C: approximately 0 to 0.30 wt%, Co: balance (in this composition, due to the fact that Ni is unavoidably present in the starting material, a ratio of at least about 0.02 wt% and at least an amount greater than an amount on the order to 50 ppm is usually included, and the "Co: balance" is the amount of Co minus trace amounts of incidental impurities).

The technique of the present invention can be applied to alloys that unavoidably include Ni. Commercially available Ni-free Co-Cr-Mo alloys or Ni-free stainless steel alloys also include an extremely small amount or trace amount of Ni. In some cases, these alloys include up to 1 wt% of Ni, up to 0.5 wt% or Ni, or at least an amount of Ni on the order of 100 ppm, for example, but the technique of the present invention can also be applied to these alloys. The present embodiment is typically applied to alloys that unavoidably include an amount of Ni on the order of several hundred parts per million, or alloys that include lesser amounts of Ni.

In the present invention, an element that forms a compound with Ni on a two-dimensional state diagram and has minimal bio-toxicity is added to a starting material that yields a composition that constitutes the abovementioned Co-Cr-Mo alloy (or Ni-free stainless steel), and the alloy composition thus obtained can be subjected to a common alloy preparation method to create an alloy. The additive element may be selected from elements that have a strong disposition towards bonding with Ni. Various types of elements that bond with Ni are known in hydrogen storage alloys (compounds), and the element used in the present invention may also be selected from elements that are known to have this characteristic. The additive element is Zr ot Ti. The element is preferably added after oxygen present in the molten metal is purged by a common deoxidation treatment. This is to prevent the Zr or Ti from reacting with oxygen dissolved in the molten metal and forming an oxide before bonding with Ni when the oxygen concentration in the molten metal is too high. These additive elements may be added and used independently, used in various combinations, or added in the form of complexes.

The amount of the additive element added to the alloy composition may be increased according to the Ni content of the alloy, and the added amount may be set in a range that creates no substantial adverse effect on the characteristics of the resultant alloy, and that allows the desired objects to be achieved. For example, when 1 wt% of Ni is present in the alloy, elution of Ni into the body can be substantially completely suppressed by adding 0.05 wt% of Zr, and the mechanical characteristics of the alloy are not adversely affected.

The ratio of the additive element added to the alloy composition with respect to 1 wt% of Ni in the alloy is 0.0001 to 1.0 wt% of Zr, preferably 0.001 to 0.5 wt%, and more preferably 0.01 to 0.1 wt% thereof; or 0.1 to 0.5 wt% Ti.

In the present invention, the alloy starting material to which the detoxification additive element is added is mixed with the additive element as needed, and then heated and melted to create a molten alloy. Vacuum induction melting (VIM), as well as various other publicly known methods, may be used as the melting method. A partial pressure of argon gas or another inert gas may be applied in the VIM furnace during the melting process. In other methods, a covering gas that contains an inert gas or nitrogen gas may be introduced into the VIM furnace. The alloy that is melted in the presence of the inert gas or covering gas may be heated as appropriate to a prescribed temperature at which a prescribed composition is obtained, or the alloy may be maintained at a prescribed temperature. The molten alloy may then be cast into an ingot or into the desired shape and cooled without modification, or may be quenched as needed. Quenching methods include water quenching, ice water quenching, oil quenching, hot bath quenching, salt bath quenching, electrolyte quenching, vacuum quenching, air quenching, injection quenching, spray quenching, stepwise quenching, time quenching, press quenching, localized quenching, ausforging, and other methods. However, these methods are used as appropriate for the alloy. Typically used methods include water quenching and ice water quenching. An ingot can be machined into the desired shape by hot extrusion, hot rolling, hot drawing, and other methods.

The molten alloy may also be formed into a thin band, a filament, or another desired shape by melt quenching. The melt quenching method may involve fluid spinning, spinning in a rotating fluid, the Kavesh method, a twin-roll process, a single-roll process, and other processes. In the melt quenching method, the molten metal is generally injected into cooled metal rolls or a refrigerant fluid to solidify the molten metal. The cooled metal rolls are usually rotated at high speed. Various types of refrigerant fluids may be used, and no particular limitation is placed thereon insofar as the desired effect is obtained, but a fluid that includes a silicone oil, for example, may be used. Examples of the silicone oil include TSF 451-30 or TSF440 polydimethylsiloxane manufactured by Toshiba Silicone Co., Ltd., but these examples are not limiting. These silicone oils may be used singly or in various combinations thereof. It is sometimes preferable to heat the silicone oils in advance under reduced pressure in order to remove low-boiling solvents or dissolved air and other gases that are included in the normal silicone oil. Disturbance of the molten metal jet flow is preferably suppressed as much as possible in order to quench and solidify the molten metal in the silicone oil to directly fabricate a metal filament. It is therefore preferred that the molten metal jet and the silicone oil be finely balanced. Specifically, the speed difference, the difference in viscosity, and the difference in surface tension and other characteristics between the molten metal jet and the silicone oil is preferably controlled. It is particularly effective in the present invention to specify the viscosity of the silicone oil.

Spinning in a rotating fluid is a method in which a fluid layer is formed in the inside of a rotating drum by centrifugal force, and the molten metal or molten alloy is sprayed from a nozzle hole and solidified in the fluid layer to create a metal filament. This method is a technique in which water, for example, is used as a cooling medium, and the alloy is sprayed from the molten state into the rotating water cooling medium to obtain a metal filament. The Kavesh method is a method such as the one described in Japanese Patent Application Laid-Open No. 49-135820 (JP A 49-135820 (December 27, 1974)), for example, and is a technique whereby a molten material is extruded into a molten filament and passed into a fluid quenching region through a controlled gaseous interface region, and the filament and the fluid medium flow concurrently in the fluid quenching region. The cooling medium used therein is a fluid medium, and may be a pure liquid, a solution, an emulsion, or a solid-liquid dispersion. The fluid medium is preferably capable of reacting with the molten material to form a stabilizing surface skin, or of being chemically non-reactive to the molten spray. The quenching medium is also preferably selected in relation to the heat capacity of the molten spray, wherein the quenching fluid is colder and/or the specific heat, density, heat of vaporization, and thermal conductivity thereof are higher when the molten spray has a large heat capacity. Other preferred general qualities of the fluid quenching medium are low cost, non-cohesive properties, non-toxicity, optical transparency, and low viscosity that minimizes disruption of the molten spray. Actually, water, a 23 wt% aqueous solution of sodium chloride at -20°C, a 21.6 wt% aqueous solution of magnesium chloride at -33°C, and a 51 wt% aqueous solution of zinc chloride at -62°C are each preferred, but it is also possible to use a silicone quenching fluid or the like such as Dow Corning 510 fluid having a viscosity level of 50 centistokes at 0 to 100°C.

The cooled alloy may be machined as appropriate. For example, the thin band, filament, or the like obtained by melt quenching and other methods may be smoothed or otherwise processed as needed for use in a medical device. The alloy may also be subjected to further homogenized heat treatment to remove segregations and the like. Homogenized heat treatment may be composed of heat treatment and quenching. The particular heat treatment used may be selected from methods publicly known in the field, and an electric furnace or the like, for example, may be used. In a typical case, heat treatment may be performed under reduced pressure or in a vacuum. Heat treatment is typically performed for 5 to 30 hours, preferably 8 to 24 hours, and more preferably 10 to 20 hours, for example. As a specific example, heat treatment is performed for 12 to 15 hours. The heating temperature is 1400°C or lower, typically 900 to 1350°C, preferably 1000 to 1300°C, and more preferably 1050 to 1250°C, for example, but the heating temperature is not limited to these examples insofar as the desired objects are achieved. A temperature of 1100 to 1200°C is a specific example. In homogenized heat treatment, quenching may be performed after the abovementioned heat treatment. Quenching is performed by the same methods as those described above.

In the present invention, allergic toxicity and other bio-toxicity due to Ni trace impurity in the bio-Co-Cr-Mo alloy (or Ni-free stainless steel) is neutralized by mixture with the additive element.

The present invention thus provides a method for neutralizing nickel toxicity of a bio-Co-Cr-Mo alloy (or Ni-free stainless steel), and also provides a bio-material and artificial prosthesis material manufactured from a Co-Cr-Mo alloy (or Ni-free stainless steel alloy) in which Ni bio-toxicity is neutralized, and an alloy thereof.

In the Co-Cr-Mo alloy of the present invention, it is possible to obtain a product in which internal defects are eliminated by adjusting the thermal history. The thermal history adjustment treatment is aimed at creating a uniform structure by collapsing shrinkage cavities, air bubbles, and the like in the forged alloy by forging, breaking down dendrite structures, and then performing recrystallization annealing. Suppression of the growth of deposits can be anticipated by quench casting using a water-cooled copper casting mold in structure adjustment. Fine dispersion of deposits, intermetallic compounds, and other secondary phases can be anticipated through high-temperature forging and other plastic processes. The suppressing effect that quenching has on growth of deposits during casting is significant when reducing the temperature at a rate of 1000°C/minute or more from the casting temperature to 400°C. Dendrites and other casting structures are disrupted by high-temperature forging, and a matrix is formed that is composed of uniaxial crystal particles that are refined to a size of 50 $\mu$m or less. Refining of the matrix is also effective for enhancing wear resistance.

The present invention also makes it possible to prevent the formation of σ phases by selecting the heat treatment method and machining temperature. Specifically, the temperature of high-temperature forging can be set to a range of 1100 to 1400°C in the system of the present invention. Even when the high-temperature-forged alloy is brought to room temperature, the formation of σ phases can be prevented through the use of water cooling and other quenching, and granular deposits or crystals can be finely dispersed in the matrix without the formation of secondary phases.

The alloy of the present invention can be made into a form that is suitable in a medical device by subjecting the alloy to such a metal gas atomization method as the one disclosed in Japanese Patent Application Laid-Open No. 62-80245 (JP A 62-80245 (April 13, 1987)), and applying the technique disclosed in Japanese Patent Application Laid-Open No. 5-1345 (JP A 5-1345 (January 8, 1993)) that utilizes the mechanical alloying method disclosed in the specification of US Patent No. 3,591,362 (US A 3591362). For example, an artificial prosthesis material can be manufactured by a process in which the alloy of the present invention that includes an additive element for Ni detoxification is made into a powder by gas atomization, the powder thus obtained is compressed by thermo-mechanical processing into a solid alloy, and the product is forged and machined as needed. The thermo-mechanical processing may include hot extrusion, hot rolling, hot pressing, and the like. The forged alloy may also be subjected to cold rolling, mechanical processing, and other processing. The manufactured product may then be machined and finished with a smooth surface, and the smooth surface may also be processed as needed to create a porous coating.

A bio-material, an artificial prosthesis material, and other medical devices can be manufactured from the Co-Cr-Mo alloy of the present invention in which Ni toxicity is neutralized. Such medical devices include bridges, dental roots, and other dental materials; prosthetic materials such as artificial bones, and surgical implants and the like, as well as biologically applicable implants, joint implants, medical artificial implants, and the like. Implant materials include artificial hip joint, artificial knees, artificial shoulders, artificial ankles, artificial elbows, other artificial joint implants, and the like. The alloy of the present invention can also be used to manufacture a member for stabilizing a bone fracture region. Such members include nails, threaded nails, nuts, screws, plates, pins, hooked pins, hooks, fixtures, bases for implants, and the like.

The technique for neutralizing bio-toxicity due to Ni in an alloy according to the present invention is also applicable to and useful for a nickel-free stainless steel alloy or a simple nickel-free stainless steel. An austenite-based nickel-free stainless steel having dramatically enhanced corrosion resistance and mechanical strength can be produced by adding nitrogen instead of nickel to a ferrite-based stainless steel in particular, and the present invention can be applied to such a nickel-free stainless steel as well. A typical example of a nickel-free stainless steel alloy is disclosed in R. C. Gebau and R. S. Brown: Adv. Mater. Process., 159 (2001) 46-48 and other publications, and such a typical alloy composition is described below.

Cr: 19.0 to 23.0 wt%, Mn: 21.0 to 24.0 wt%, Mo: 0.5 to 1.5 wt%, $N_2$: 19.0 to 23.0 wt%, Fe: balance.

In this composition, due to the fact that Ni is unavoidably present in the starting material, a ratio on the order of 100 ppm to 1.0 wt% of Ni is usually included, and the "Fe: balance" is the amount of Fe minus trace amounts of incidental impurities.

The description given above on the basis of the abovementioned Co-Cr-Mo alloy can also be applied to the nickel-free stainless steel alloy (however, it is apparent to one skilled in the art that the processing temperatures and other parameters are modified with consideration for the differences in melting point and other properties with respect to the abovementioned Co-Cr-Mo alloy). The nickel-free stainless steel alloy may be caused to absorb nitrogen after being subjected to processing prior to the addition of nitrogen, and made into the desired material/product. For example, it is known that a molded article can be caused to absorb about 1 wt% of nitrogen to create an austenite by bringing the molded article into contact with nitrogen gas in a heated heat-treatment furnace.

The present invention provides a technique for suppressing ion elution in a biological environment that is based on the fact that the ion elution rate of c phases is significantly slower than that of γ phases, which was discovered as a result of detailed investigation of the ion elution behavior of γ phases and ε phases that occur in a bio-Co-Cr-Mo alloy, e.g., Co-29Cr-6Mo alloy. The fact that ion elution rates vary according to differences in crystal structures that occur in an alloy had not yet been discovered, and this fact was understood for the first time in the present invention. The present invention thus provides a technique for suppressing allergic reaction through the use of a technique for controlling the structure of a Co-Cr-Mo alloy, e.g., Co-29Cr-6Mo alloy, whereby an ε phase, which is a crystal structure having a low ion elution rate, is actively utilized to reduce the rate of ion elution from the surface of a Co-Cr-Mo alloy, e.g., Co-29Cr-6Mo, implanted in a body.

The basic structure of an ASTM F75 alloy proven as a bio-Co-Cr-Mo alloy is Co-29Cr6Mo (it should be noted, of course, that the elements herein have certain allowable ranges such as the ones described above), but a maximum of 0.35% of C and 1% of Ni are included therein. The micro-structure of the Co-29Cr-6Mo alloy is composed of FCC (Face-Centered Cubic) phases and carbide phases. It is also common for a small amount of HCP phases (ε phases) to be included in addition to the FCC phases (γ phases) and carbide phases. HCP (Close-Packed Hexagonal) phases are c phases (ε martensite phases) formed by rapid cooling after melting or heat treatment at a high temperature of 1000°C or higher. Alternatively, the HCP phases are ε phases (massive ε phases) deposited from diffused transformation by long-term heat treatment in a temperature range of about 1000°C to about 600°C.

It may be assumed in the present invention that the prescribed structure control technique/ion elution control technique may be applied without limitation insofar as the bio-Co-Cr-Mo alloy is known, and the techniques of the present invention may be applied to the abovementioned Co-Cr-Mo alloy, for example. The Co-Cr-Mo alloy also includes an alloy composition such as the one described below, for example.

Cr: 25.0 to 31.0 wt%, preferably 26.0 to 30.0 wt%, and more preferably 28.0 to 29.5 wt%;

Mo: 4.0 to 8.0 wt%, preferably 5.0 to 7.0 wt%, and more preferably 5.5 to 6.5 wt%; and

Co: balance.

In this composition, due to the fact that Ni is unavoidably present in the starting material, a ratio of at least 0.2 to 1.0 wt% of Ni is usually included, and the "Co: balance" is the amount of Co minus trace amounts of incidental impurities. C, Fe, Si, $N_2$, and other trace elements may also be included.

Carbon has been included in a maximum ratio of 0.35% until now in ASTM F75 alloys for the purpose of forming carbides. A maximum amount of 1% or nickel is also allowed as a trace impurity. Since these elements have the effect of increasing the stacking fault energy of the Co-Cr-Mo alloy, $\gamma$ phases are stabilized, and the constituent phases obtained are $\gamma$ phases and carbide phases. In some cases, a small amount of $\epsilon$ phases (martensite phases) formed by rapid cooling after melting or heat treatment at a high temperature of 1000°C or higher are included in addition to the $\gamma$ phases and carbide phases.

In contrast, the amount of added carbon is controlled in the present invention to the extent that $\gamma$ phases are not deposited, and the deposition ratio of $\epsilon$ phases is increased. The ion elution rate can thereby be reduced. The ion elution rate can also be reduced by causing massive $\epsilon$ phases to be deposited by maintaining the alloy in a temperature range of about 600°C from a temperature lower than about 1000°C.

The present invention provides a method for suppressing ion elution from a bio-Co-Cr-Mo alloy by adjusting the alloy structure in controlled fashion to enrich the bio-Co-Cr-Mo alloy with an $\epsilon$ HCP phase structure. Adjusting the alloy structure in the bio-Co-Cr-Mo alloy in controlled fashion can be achieved by (1) adding an element or compound selected from the group that includes elements in groups 4, 5, and 13 of the periodic table, lanthanide elements, misch metals, and Mg to the alloy composition and/or (2) performing appropriate heat treatment. The additive element is be selected from the group that includes zirconium and titanium. The additive element is preferably zirconium. The added amount may be adjusted so that the desired effects are obtained as described above, or selected from such ranges as those described above. Control of the Co-Cr-Mo alloy structure may include performing heat treatment at a temperature of 600°C to 1250°C after alloy melting. The control of the Co-Cr-Mo alloy structure may also include (i) melting an alloy composition or heat treating an alloy composition at a temperature of 1000°C or higher, and then rapidly cooling the alloy composition; or (ii) heat treating an alloy composition for a long period of time at a temperature of approximately 1000°C or higher and in a temperature range of at least 550 to 650°C. Suppression or reduction of ion elution from the alloy in the present invention may denote a reduction in the ion elution rate according to testing in the present specification in comparison to currently available alloys or alloys of nominal composition, e.g., Co-29Cr-6Mo alloy. The degree of reduction may refer to a reduction in individual ions, a reduction in all ions, a reduction in ions that are more conducive to bio-toxicity, or other reductions.

The present invention provides a novel Co-Cr-Mo alloy in which an alloy structure in the bio-Co-Cr-Mo alloy is enriched with an $\epsilon$ HCP phase structure, and ion elution from the alloy is suppressed or reduced. The alloy may be one in which heat treatment at a temperature of 600°C to 1250°C is performed after alloy melting to cause enrichment with an $\epsilon$ HCP phase structure. The alloy may also be one in which (i) an alloy composition is melted or heat treated at a temperature of 1000°C or higher, and then rapidly cooled; or (ii) an alloy composition is heat treated for a long period of time at a temperature of approximately 1000°C or higher and in a temperature range of at least 550 to 650°C to cause enrichment with an $\epsilon$ HCP phase structure. The present invention provides a medical device manufactured from a bio-Co-Cr-Mo alloy that is enriched with an $\epsilon$ HCP phase structure, and in which ion elution from the alloy is suppressed or reduced. As described above, this device may be manufactured by subjecting the Co-Cr-Mo alloy to a process selected from the group that includes quenching, metal gas atomization, mechanical alloying, liquid quenching, hot extrusion, hot rolling, hot drawing, and forging.

The alloy (Co-Cr-Mo alloy, e.g., Co-29Cr-6Mo alloy) obtained by structure control in the present invention (including cases of structure control by control of the additive element) can thus be applied as a bio-material having minimal bio-toxicity, i.e., increased safety, in artificial hip joints, stents, and various other medical devices. It must be understood that the techniques/processes/ applications and the like described in relation to the abovementioned "Co-Cr-Mo alloy in which nickel toxicity is neutralized" may be applied in the same manner to the present structure-controlled alloy.

The "periodic table" referred to in the present specification is in accordance with the notation system employed in conjunction with the revision of IUPAC (International Union of Pure Applied Chemistry) inorganic chemical naming conventions in 1989.

Elements in group 4 of the periodic table include Ti, Zr, Hf, and the like. Elements in group 5 of the periodic table include V, Nb, Ta, and the like. Elements in group 13 of the periodic table include B, Al, Ga, In, Tl, and the like.

Examples

The present invention will be described hereinafter using specific examples, but the examples are provided merely for reference to specific embodiments in order to describe the present invention. These examples are given to describe specific embodiments of the present invention, but do not limit or restrict the scope of the invention disclosed in the present application. It is apparent that various embodiments of the present invention are possible based on the idea of the present specification.

All of the examples have been or can be implemented using standard techniques unless otherwise specified, and these techniques are known to and commonly used by those skilled in the art.

Example 1

(Search For Additive Element X For Fixing Trace Amounts of Ni)

Elements that form compounds with Ni and have low bio-toxicity were searched for on a two-dimensional state diagram. As a result, aluminum (Al), titanium (Ti), zirconium (Zr), and niobium (Nb) were selected as potential additive elements X.

(Sample Composition and Sample Melting)

The sample composition is described below. Al: 0.5 wt%, Ti: 0.3 wt%, Zr: 0.05 wt%, and Nb: 0.5 wt% were each added to a composition of Co: balance, Cr: 29 wt%, Mo: 6 wt%, and Ni: 1 wt% as a control. In this sample composition, 1 wt% of Ni was intentionally included to facilitate comparison of the amount of Ni elution. The sample was melted using a high-frequency vacuum induction melting furnace. Carbon was added in a state in which the molten sample was maintained in a vacuum, and additive element X was added after thorough deoxygenation.

(Test Sample)

An alloy sample for testing was fabricated by a melt forging apparatus.
The fabricated sample was machined to a size of $10 \times 30 \times 1$ mm$^3$ by a wire cutting electric discharge machine, and homogenized heat treatment (1150°C, 12 hours, followed by water quenching: W. Q.) was performed to completely remove segregations. The test sample was ground to SiC No. 1000 in distilled water, then thoroughly rinsed in acetone and distilled water for 5 minutes by ultrasonic cleaning, and allowed to stand for 24 hours or longer in air to form an atmospheric coating.

(Testing Method)

In the test solution, (1 + 99) lactic acid (1% aqueous solution of lactic acid) was used for accelerated testing in order to better ascertain the elution rate of each metal and facilitate comparison of the alloy test samples.

Two test samples were placed in a test vessel so as not to overlap each other, and the test samples were completely immersed in the test solution. The volume of the test solution was 30 mL. The same test was performed using only the test solution with no sample placed therein (blank experiment). The elution conditions were a solution temperature of $37 \pm 1$°C, a test period of 7 days, and a static condition as a basis.

(Measurement of Test Solution Concentration)

The test sample was removed after testing was completed, the test sample and the inside of the vessel were rinsed with (1 + 99) nitric acid (1% aqueous solution of nitric acid), the contents were filtered, and the analysis solution was standardized (100 mL). The metal concentration was then measured by ICP emission spectrochemical analysis, and the elution rate of each metal was calculated using the equation below.

$$W_i = L(IC_i - IB_i)/S$$

In the equation, $W_i$ : elution rate (g/cm$^2$) per unit surface area of i element
$IC_i$: concentration (g/mL) of i element being eluted after elution test
$IB_i$: average concentration (g/mL) of i element in blank test solution
L: total quantity (mL) of elution test solution
S: overall surface area (cm$^2$) of test sample.

(Test Results)

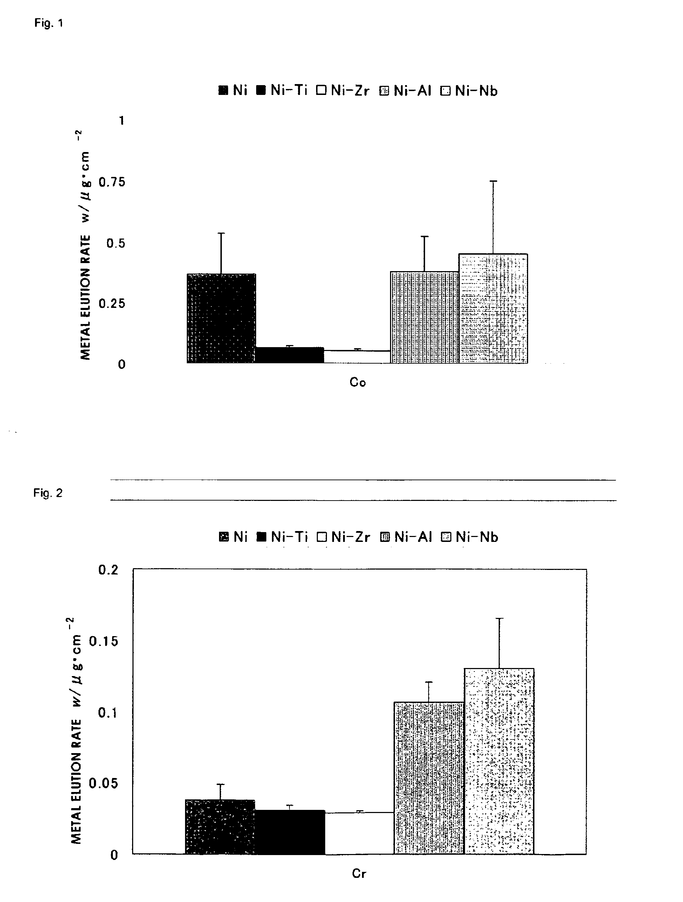

The results of testing metal elution are shown in FIGS. 1 through 5.
FIG. 1 shows the rates of Co metal elution in 1% lactic acid. Using Ni not included in the additive element as the control, the alloys to which Al and Nb were added showed substantially the same rate of Co metal elution as the control. In comparison, the alloys to which Ti and Zr were added had about 1/5 the Co metal elution rate of the control.
FIG. 2 shows the rates of Cr metal elution in 1% lactic acid. The elution rates from the alloys to which Ti and Zr were added were the same as the rate of Cr metal elution from the control. The elution rates of Cr metal from the alloys to which Al and Nb were added was three times higher or more than the elution rate of the control.

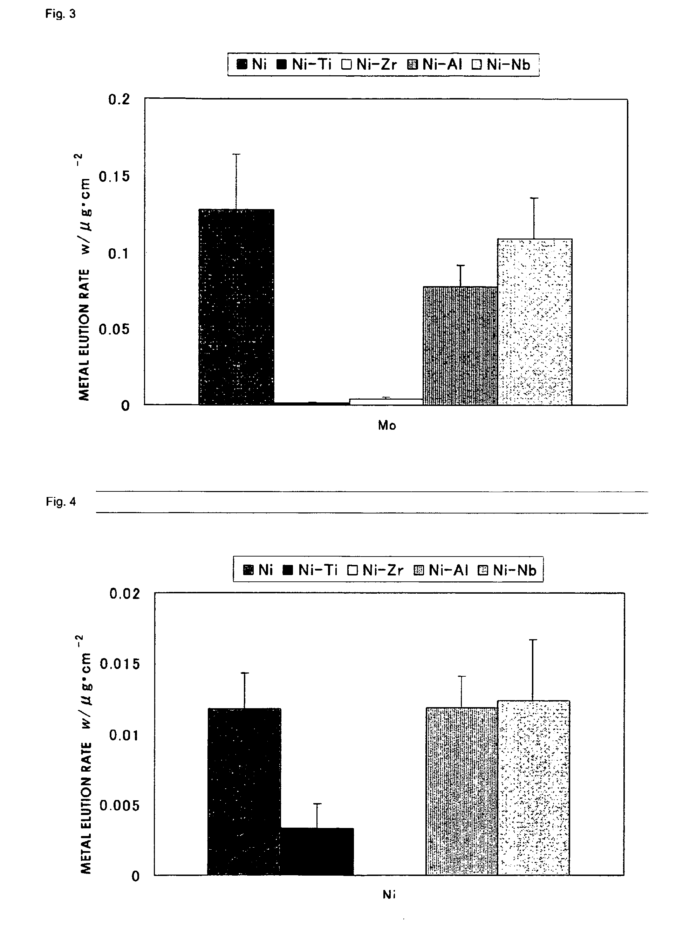

FIG. 3 shows the rates of Mo metal elution in 1% lactic acid. The elution rates were less than the elution rate of the control in the alloy samples of all of the additive elements. The Mo metal elution rates were slightly lower in the alloys to which Al and Nb were added, and the Mo metal elution rates were 1/10 or less that of the control in the alloys to which Ti and Zr were added.
FIG. 4 shows the rates of Ni metal elution in 1% lactic acid. The Ni elution rates in the alloys to which Al and Nb were added were substantially the same as in the control. The Ni elution rate in the alloy to which Ti was added was ¼ or less of the Ni elution rate of the control, and absolutely no Ni was eluted in the alloy to which Zr was added.

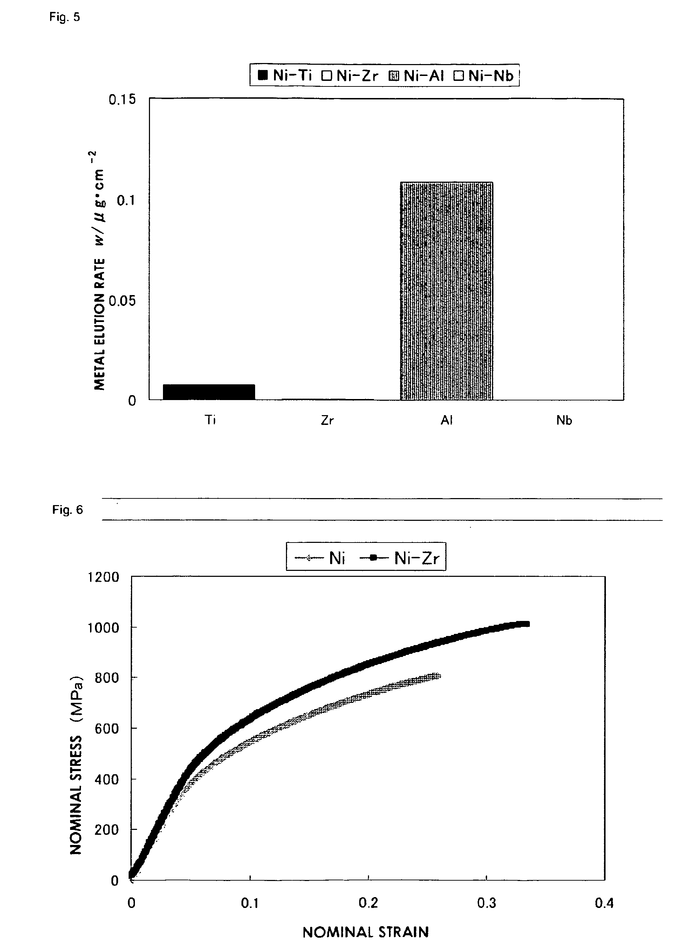

FIG. 5 shows the metal elution rates of each additive element in 1% lactic acid. The elution rate for Al was extremely high. In contrast, the elution rates of Ti and Zr were extremely low, and there was no elution of Nb.

(Summary of Elution Testing)

Suppressing effects on Ni metal elution was not observed in the alloys in which 0.5Al and 0.5Nb were added to Co-29Cr-6Mo-1Ni. Not only were suppressing effects on Ni metal elution observed with regard to alloys in which 0.3Ti and 0.05Zr were added to Co-29Cr-6Mo-1Ni, but elution suppressing effects were observed with respect to Co and Mo besides Ni. Elution of Ni was not observed in the alloy to which 0.05Zr was added in particular.

It is apparent from these results that elution of Ni can be suppressed, i.e., Ni can be fixed, through addition of trace amounts of Ti and Zr.

(Tensile Test Results)

Metal elution was suppressed most significantly in the alloy to which a trace amount of Zr was added. However, the effects of trace addition on mechanical characteristics are not known. The mechanical characteristics of the alloy to which a trace amount of Zr was added were therefore compared to those of the control.

FIG. 6 shows the nominal stress/nominal strain curves for Co-29Cr-6Mo-1Ni alloy and Co-29Cr-6Mo-1Ni-.05Zr alloy. The alloy to which Zr was added had a stress at break of 1011 MPa, which was higher than the stress at break of 807 MPa of the control sample. The yield stress and the plastic elongation of the control samples were 335 MPa and 16.5%, respectively, whereas these values were 420 MPa and 23.0% in the alloy to which Zr was added. It was apparent that the mechanical characteristics were significantly enhanced by the addition of a trace amount of Zr. These results are considered to show that adding a trace amount of Zr to the alloy does not have the effect of reducing mechanical characteristics.

Example 2

The test sample composition was as described below.

Lanthanide elements, i.e., La, Ce, Pr, Nd, Pm, Sm, Eu, Gd, Tb, Dy, Ho, Er, Tm, Yb, and Lu, and a misch metal, i.e., La-Ce misch metal, were each added to a control having the composition Co: balance, Cr: 29wt%, Mo: 6wt%, Ni: 1wt%. In the same manner as in Example 1, 1 wt% of Ni was intentionally included in the sample composition to facilitate comparison of the amount of Ni elution. The same processing as that of Example 1 can be performed to confirm the suppression of Ni elution, i.e., the fixing of Ni. The basis for the Ni fixing observed in Example 1 is the addition of an element having a strong disposition to bond to Ni. When elements that bond to Ni are searched for, numerous such elements are identified among hydrogen storage alloys (compounds), and hydrogen storage alloys that form compounds with Ni are thus found among lanthanide elements. The same effects as those obtained by addition of Zr can therefore be anticipated. The same operation as that of Example 1 can also be performed for alloys in which Ni is present in the composition Fe-(19-23)Cr-(21-24)Mn-(0.5-1.5)Mo-(0.85-1.1)N to confirm the suppression of Ni elution, i.e., the fixing of Ni.

An MgO crucible is used to prepare an alloy sample having the same control alloy sample composition. The melt temperature is maintained at 1600°C or higher, whereupon the molten metal and the MgO crucible react, and Mg begins to melt into the alloy. The melted Mg and the Ni bond together, and the Ni is fixed. The product of the bonding between Mg and Ni floats to the surface of the molten metal due to specific gravity, and is removed as slag, and removal of Ni occurs. This result can also be obtained by adding Mg to the molten metal using a common alumina crucible.

Example 3

The test sample composition was as described below.

In the same manner as in Example 1, 1 wt% of Ni was intentionally included in the following sample composition to facilitate comparison of the amount of Ni elution: Co: balance, Cr: 29 wt%, Mo: 6wt%, Ni: 1 wt%. A sample of the composition was fabricated by forging using an alumina ($Al_2O_3$) crucible and a magnesia (MgO) crucible. Casting was performed by a method in which the melt temperature was maintained temporarily at 1600°C to 1650°C or above, after which carbon was added to the molten metal while still in a vacuum, the oxygen blended into the molten metal was removed, the melt temperature was decreased to 1400 to 1450°C and maintained for a short time, and the molten metal was cast into a die. The sample thus fabricated was subjected to elution testing by the same process as described in Example 1.

(Test Results)

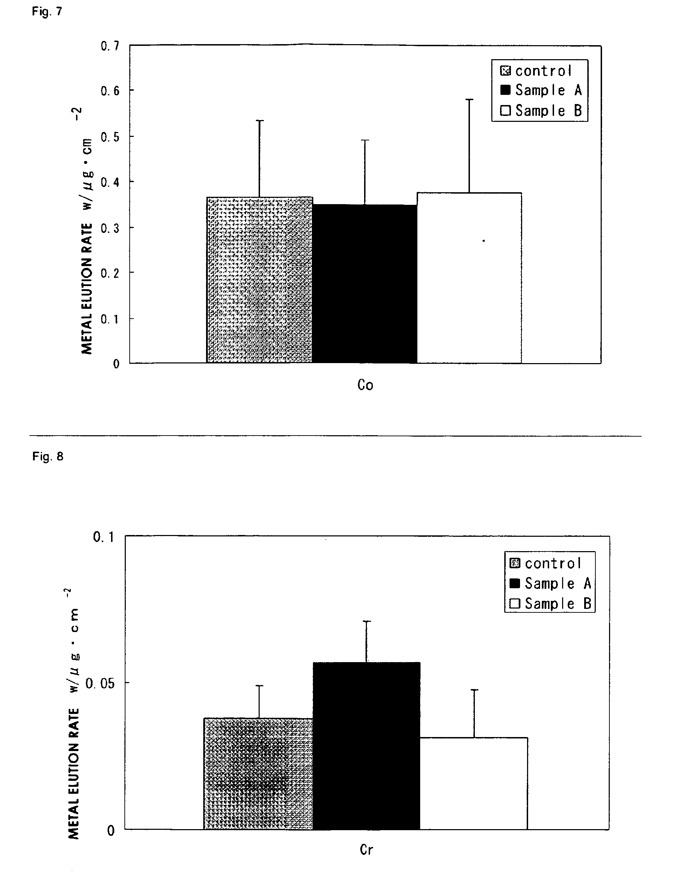

The results of testing metal elution are shown in FIGS. 7 through 10. The Co-29Cr-6Mo-1Ni alloy used in Example 1 was the control, the alloy fabricated in the $Al_2O_3$ crucible was designated as Sample A, and the alloy fabricated in the MgO crucible was designated as Sample B.

FIG. 7 shows the rates of Co metal elution in 1% lactic acid. The alloys of Sample A and Sample B both had substantially the same Co metal elution rate as the control.

FIG. 8 shows the rates of Cr metal elution in 1% lactic acid. Sample A had a slightly higher elution rate than the control, and Sample B had a slightly lower elution rate than the control, but the elution rates had comparable levels.

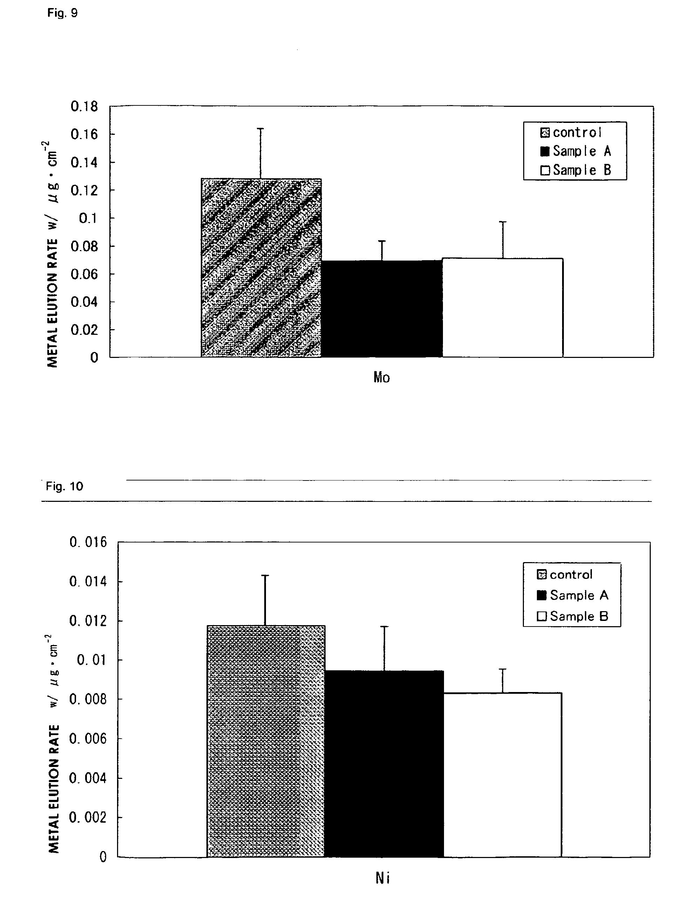

FIG. 9 shows the rates of Mo metal elution in 1% lactic acid. The elution rates of Mo metal in both Sample A and Sample B were approximately ½ the metal elution rate of the control.

FIG. 10 shows the rates of Ni metal elution in 1% lactic acid. The elution rates of Ni metal in both Sample A and Sample B were lower than the control. The suppressing effect was greater in Sample B than in Sample A.

(Summary)

When the melt temperature is maintained at 1600°C or higher using an $Al_2O_3$ crucible and an MgO crucible, the molten metal and the crucibles react, and Al or Mg melt into the molten metal. The Al and Mg that seeps into the molten metal bond with Ni, and the Ni is fixed. The product of the bonding between the Al, Mg, and Ni floats to the surface of the molten metal due to specific gravity, and is removed as slag, and removal of Ni occurs. It is inferred that the elution rate is reduced as a result.

The test results confirmed a reducing effect on the Ni elution rate, but results equivalent to those of the alloy to which Zr was added in Example 1 were not obtained. However, adequate suppression of Ni elution was obtained by performing the appropriate heat treatment/ machining after casting.

Example 4

An element for increasing the stacking fault energy was added to Co-Cr-Mo alloy, γ phases in the alloy structure were stabilized, and the rate of ion elution from the resultant alloy was investigated.

(Method)

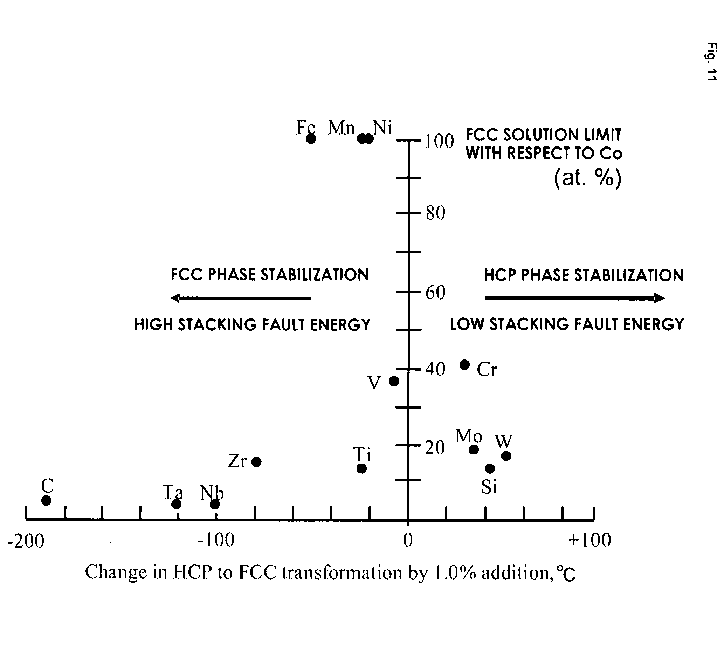

FIG. 11 shows the effects of the additive element on the HCP-to-FCC phase transformation temperature (Ms) of Co, wherein the vertical axis indicates the solution limit of the additive element, and the horizontal axis indicates the change in the Ms temperature due to addition of 1.0% of the additive element(C. T. Sims, N. S. Stoloff & W. C. Hagel, SUPERALLOYS II, Wiley-Interscience (1987)). As the minus temperature increases from 0 (to the left in FIG. 11), the Co stacking fault energy increases, and the FCC crystal stabilizing effects increase (left side). Conversely, as the plus temperature increases from zero (to the right in FIG. 11), the Co stacking fault energy decreases, and HCP crystals are stabilized (right side).

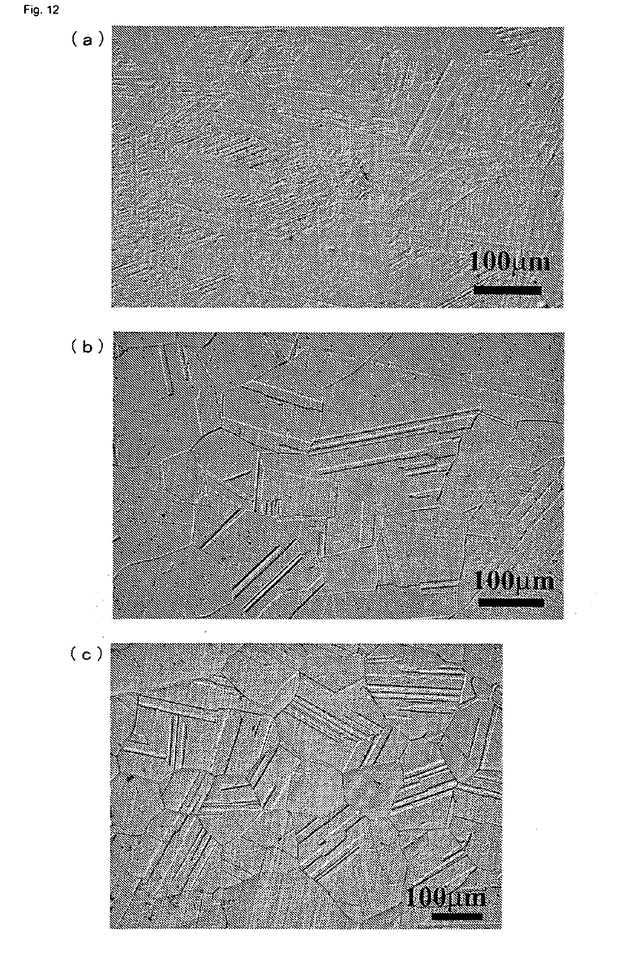

The elements Nb and Zr were selected based on FIG. 11 as elements for stabilizing γ phases, and an alloy in which 0.3 wt% of Nb and 0.1 wt% of Zr were added to Co-29 wt%Cr-6 wt%Mo-1 wt%Ni alloy (referred to as "non-additive material," wherein the comparison material is the nominal composition: Co: balance, Cr: 29 wt%, Mo: 6 wt%, Ni: 1 wt%. An amount of 1 wt% of Ni is intentionally added) was formed into an ingot using an argon arc melting furnace. FIG. 12 shows the structure of the alloy that was water-quenched after being maintained at a temperature of 1150°C for 12 hours. The structure was observed by optical microscope after the test samples were polished to a mirror finish using 0.3 μm particles of $Al_2O_3$ and subjected to electrolytic polishing in methanol sulfate (the structure was observed in the same manner for other alloys).

In the optical microscope structure of the non-additive material shown in FIG. 12A, besides flat structures that correspond to γ phases, numerous fine straight-lined structures (striations) that correspond to c martensite phases are observed. The structures of the Nb and Zr-additive materials shown in FIGS. 12B and 12C are almost all changed to y phase flat structures. It was confirmed from these results that the addition of Nb and Zr stabilizes γ phases.

Ion elution testing was then conducted using the method described below.

An ingot of an alloy in which 0.3 wt% of Nb or 0.1 wt% of Zr was added to Co-29 wt%Cr-6 wt%Mo-1 wt%Ni alloy was formed using an argon arc melting furnace. The test sample of the alloy, which was maintained at a temperature of 1150°C for 12 hours and water-quenched, was ground to SiC No. 1000 in distilled water, then thoroughly rinsed in acetone and distilled water for 5 minutes by ultrasonic cleaning, and allowed to stand for 24 hours or longer in air to form an atmospheric coating.

(Elution Testing Method)

In the same manner as Example 1, (1 + 99) lactic acid was used in the test solution for accelerated testing in order to better ascertain the elution rate of each metal and facilitate comparison of the alloy test samples. Two test samples were placed in a test vessel so as not to overlap each other, and the test samples were completely immersed in the test solution. The volume of the test solution was 30 mL. The same test was performed using only the test solution with no sample placed therein (blank experiment).

The elution conditions were a solution temperature of 37±1°C, a test period of 7 days, and a static condition as a basis.

(Measurement of Test Solution Concentration)

In the same manner as Example 1, the test sample was removed after testing was completed, the test sample and the inside of the vessel were rinsed with (1 + 99) nitric acid, the contents were filtered, and the analysis solution was standardized (100 mL). The metal concentration was then measured by ICP emission spectrochemical analysis, and the elution rate of each metal was calculated using the equation below.

$$W_i = L(IC_i - IB_i)/S$$

In the equation, $W_i$: elution rate (g/cm$^2$) per unit surface area of i element
$IC_i$: concentration (g/mL) of i element being eluted after elution test
$IB_i$: average concentration (g/mL) of i element in blank test solution
L: total quantity (mL) of elution test solution
S: overall surface area (cm$^2$) of test sample.

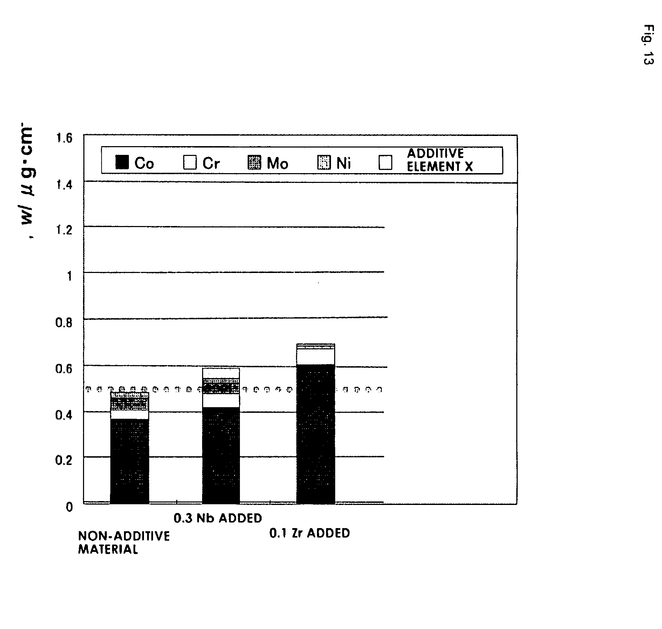

The results of ion elution testing are shown in FIG. 13. The total quantity of eluted elements in the alloys to which Nb and Zr were added, which had a high ratio of $\gamma$ phases, is larger than that of the non-additive alloys, which had a large ratio of $\varepsilon$ phases deposited in addition to $\gamma$ phases. A tendency for the elution rate of Ni ions to be reduced was identified in the Zr-added alloy.

Example 5

The amount of Zr, which is an element for increasing the stacking fault energy, added to the Co-Cr-Mo alloy was increased from 0.05 wt% to 0.3 wt%, and the degree of $\gamma$ phase stabilization was varied. The rate of ion elution from the resultant alloy was investigated.

(Method)

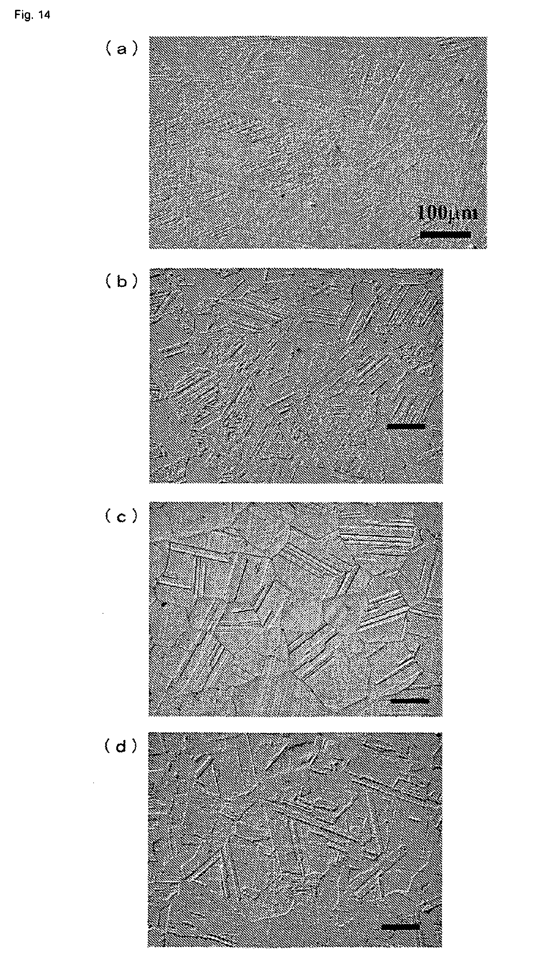

Ingots of an alloy in which 0.05, 0.1, and 0.3 wt% of Zr was added to Co-29 wt%Cr-6 wt%Mo-1 wt%Ni alloy was formed using an argon arc melting furnace. FIG. 14 shows the structure of the alloy maintained at 1150°C for 12 hours and water-quenched.

In the optical microscope structure of the non-additive material shown in FIG. 14A, besides flat structures that correspond to $\gamma$ phases, numerous fine straight-lined structures (striations) that correspond to $\varepsilon$ martensite phases are observed. The surface area fraction of the striations is higher in FIG. 14B than in the non-additive material when 0.05 wt% of the element is added. The reason for this is considered to be that because the amount of added Zr was extremely small, compounds were formed with Ni and other trace elements, and the Zr was consumed. Therefore, the stacking fault energy may be somewhat reduced in comparison to that of the alloy to which Zr was not added. The stacking fault energy may increase when more Zr is added. The amount of added Zr is increased to 0.1 and 0.3 wt% in FIGS. 14C and 14D. The surface area fraction of the striations is correspondingly reduced. The increase in the ratio of $\gamma$ phases was apparent from the results of X diffraction testing.

(Results)

Ion elution testing was then performed by the same method as the one used in Example 4.
FIG. 5 shows the results of ion elution testing for each test material. Since the ratio of $\varepsilon$ phases was high in the alloy to which 0.05% of Zr was added, the ion elution rate was low compared to the non-additive alloy. Adding 0.1 and 0.3% of Zr increased the stacking fault energy and stabilized the $\gamma$ phases. There was also a corresponding increase in the ion elution rate.
To summarize the results of Examples 4 and 5, it was learned that metal elution can be suppressed by separating $\varepsilon$ HCP structures from $\gamma$ FCC structures.

INDUSTRIAL APPLICABILITY

The present invention makes it possible to provide a Co-Cr-Mo alloy and a Ni-free stainless steel having excellent corrosion resistance, wear resistance, and biological compatibility while suppressing or preventing the occurrence of nickel bio-toxicity by adding zirconium or another additive element. Since the present invention makes it possible to neutralize nickel toxicity by an inexpensive and simple method, the resultant alloy is economically advantageous and can be utilized in a wide range of applications, e.g., manufacturing biologically compatible materials or medical devices. The present invention provides a technique for controlling the structure of Co-Cr-Mo alloy to form a crystal structure having a low rate of ion elution. A technique is thus provided for reducing the rate of ion elution from the surface of a Co-Cr-Mo alloy implanted in the body by enriching the Co-Cr-Mo alloy with $\varepsilon$ phases that are crystal structures having a low ion elution rate, suppressing allergic reactions, and suppressing or preventing other occurrences of bio-toxicity. This technique can be utilized in a wide range of applications, e.g., manufacturing biologically compatible materials or medical devices.
It is apparent that the present invention may be implemented in ways other than those mentioned in the description and specified in the examples. Numerous improvements and modifications of the present invention can be made using the information contained in the description above, and are accordingly encompassed in the range of the attached claims.

## Claims

1. A method for neutralizing bio-toxicity due to Ni trace impurity in a bio-Co-Cr-Mo alloy or a Ni-free stainless steel alloy in which Ni is unavoidably present; said method for neutralizing nickel toxicity of the bio-Co-Cr-Mo alloy or Ni-free stainless steel alloy **characterized in** comprising adding an element selected from Zr or Ti to the alloy composition, whereby the additive element is added after oxygen present in the molten metal is purged and whereby the ratios of the additive element added to the alloy composition are 0.0001 to 1.0 wt% of Zr or 0.1 to 0.5 wt% of Ti per 1 wt% of Ni.

2. The method for neutralizing nickel toxicity according to claim 1, **characterized in that** the nickel content in the alloy composition is (1) about 1.0 wt% or less, (2) about 0.5 wt% or less, (3) about 0.002 wt% or less, (4) at least on the order of 100 ppm or less, or (5) on the order of several hundred parts per million or less.

3. The method for neutralizing nickel toxicity in a bio-Co-Cr-Mo alloy or a Ni-free stainless steel alloy according to claims 1 or 2, **characterized in** performing heat treatment at a temperature of 600°C to 1250°C after alloy melting.

4. A bio-Co-Cr-Mo alloy or Ni-free stainless steel alloy wherein Ni is unavoidably present in the alloy and In which nickel toxicity is neutralized obtained by the method according to claim 1, **characterized in** comprising the bio-Co-Cr-Mo alloy or Ni-free stainless steel alloy in which an element selected from Zr or Ti is added to the alloy composition in order to neutralize bio-toxicity due to Ni trace impurity, whereby the ratios of the additive element added to the alloy composition are 0.0001 to 1.0 wt% of Zr or 0.1 to 0.5 wt% of Ti per 1 wt% of Ni.

5. The alloy according to claim 4, **characterized in** comprising the alloy having a nickel content of (1) about 1.0 wt% or less, (2) about 0.5 wt% or less, (3) about 0.002 wt% or less, (4) at least on the order of 100 ppm or less, or (5) on the order of several hundred parts per million or less.

6. The bio-Co-Cr-Mo alloy or Ni-free stainless steel alloy in which nickel toxicity is neutralized according to claims 4 and 5, **characterized in that** heat treatment at a temperature of 600°C to 1250°C Is performed after alloy melting.

7. A medical device manufactured from the bio-Co-Cr-Mo alloy or Ni-free stainless steel alloy in which nickel toxicity is neutralized according to claim 4.

8. A medical device manufactured by subjecting the bio-Co-Cr-Mo alloy or Ni-free stainless steel alloy In which nickel toxicity is neutralized according to claim 4 to a process selected from the group that includes quenching, metal gas atomization, mechanical alloying, liquid quenching, hot extrusion, hot rolling, hot drawing, and forging.

## Patentansprüche

1. Verfahren zum Neutralisieren der Biotoxizität aufgrund einer Ni-Spurenverunreinigung in einer Bio-Co-Cr-Mo-Legierung oder einer Ni-freien Edelstahllegierung, in welcher Ni unvermeidlich vorliegt, wobei das Verfahren zum Neutralisieren der Nickeltoxizität der Bio-Co-Cr-Mo-Legierung oder der Ni-freien Edelstahllegierung **dadurch gekennzeichnet ist, dass** es das Hinzufügen eines Elementes, ausgewählt unter Zr oder Ti, zu der Legierungszusammensetzung umfasst, wobei das zugefügte Element hinzugefügt wird, nachdem Sauerstoff, der in dem geschmolzenen Metall vorliegt, beseitigt wurde und wobei die Verhältnisse des zugefügten Elements, welches zu der Legierungszusammensetzung hinzugefügt wird, 0,0001 bis 1,0 Gew.-% Zr oder 0,1 bis 0,5 Gew.-% Ti pro 1 Gew.-% Ni betragen.

2. Verfahren zum Neutralisieren der Nickeltoxizität nach Anspruch 1, **dadurch gekennzeichnet, dass** der Nickelgehalt in der Legierungszusammensetzung (1) etwa 1,0 Gew.-% oder weniger, (2) etwa 0,5 Gew.-% oder weniger, (3) etwa 0,002 Gew.-% oder weniger, (4) mindestens in der Größenordnung von 100 ppm oder weniger oder (5) in der Größenordnung von einigen hundert ppm (parts per million) oder weniger beträgt.

3. Verfahren zum Neutralisieren der Nickeltoxizität in einer Bio-Co-Cr-Mo-Legierung oder einer Ni-freien Edelstahllegierung nach Anspruch 1 oder 2, **gekennzeichnet durch** Ausführen einer Hitzebehandlung bei einer Temperatur von 600° C bis 1250° C nach dem Schmelzen der Legierung.

4. Bio-Co-Cr-Mo-Legierung oder Ni-freie Edelstahllegierung, bei der Ni in der Legierung unvermeidlich vorliegt und in welcher die Nickeltoxizität durch das Verfahren nach Anspruch 1 neutralisiert wird, **dadurch gekennzeichnet, dass** es die Bio-Co-Cr-Mo-Legierung oder Ni-freie Edelstahllegierung aufweist, wobei ein Element, ausgewählt unter Zr oder Ti, zu der Legierungszusammensetzung hinzugefügt ist, um die Biotoxizität aufgrund einer Ni-Spurenverunreinigung zu neutralisieren, wobei die Verhältnisse des zugefügten Elements, welches zu der Legierungszusammensetzung hinzugefügt wird, 0,0001 bis 1,0 Gew.-% Zr oder 0,1 bis 0,5 Gew.-% Ti pro 1 Gew.-% Ni betragen.

5. Legierung nach Anspruch 4, **dadurch gekennzeichnet, dass** es die Legierung mit einem Nickelgehalt von (1) etwa 1,0 Gew.-% oder weniger, (2) etwa 0,5 Gew.-% oder weniger, (3) etwa 0,002 Gew.-% oder weniger, (4) mindestens in der Größenordnung von 100 ppm oder weniger oder (5) in der Größenordnung von einigen hundert ppm (parts per million) oder weniger aufweist.

6. Bio-Co-Cr-Mo-Legierung oder Ni-freie Edelstahllegierung, in welcher die Nickeltoxizität nach Anspruch 4 oder 5 neutralisiert ist, **dadurch gekennzeichnet, dass** eine Hitzebehandlung bei einer Temperatur von 600° C bis 1250° C nach dem Schmelzen der Legierung ausgeführt wird.

7. Medizinische Vorrichtung, die aus der Bio-Co-Cr-Mo-Legierung oder der Ni-freien Edelstahllegierung hergestellt ist, in welcher die Nickeltoxizität gemäß Anspruch 4 neutralisiert ist.

8. Medizinische Vorrichtung, die hergestellt ist, indem die Bio-Co-Cr-Mo-Legierung oder die Ni-freie Edelstahllegierung, in welcher die Nickeltoxizität nach Anspruch 4 neutralisiert ist, einem Prozess unterzogen wird, der ausgewählt ist unter Abschrecken, Metallgasatomisierung, mechanisches Legieren, Abschrecken mit einer Flüssigkeit, Warmstrangpressen, Warmwalzen, Warmziehen und Schmieden.

**Revendications**

1. Procédé de neutralisation de la bio-toxicité due à une impureté sous la forme de trace de Ni dans un bio-alliage de Co-Cr-Mo ou d'un alliage d'acier inoxydable sans Ni dans lequel Ni est inévitablement présent, ledit procédé de neutralisation de la toxicité, due au nickel, du bio-alliage de Co-Cr-Mo ou de l'alliage d'acier inoxydable sans Ni étant **caractérisé par** l'addition d'un élément sélectionné parmi Zr ou Ti à la composition d'alliage, dans lequel l'élément additionnel est ajouté après avoir éliminé l'oxygène présent dans le métal fondu et les taux de l'élément additionnel ajouté à la composition d'alliage sont de 0,0001 à 1,0% en poids de Zr ou de 0,1 à 0,5% en poids de Ti pour 1% en poids de Ni.

2. Procédé de neutralisation de la toxicité du nickel selon la revendication 1, **caractérisé en ce que** la teneur en nickel dans la composition d'alliage est (1) d'environ 1,0% en poids ou moins, (2) d'environ 0,5% en poids ou moins, (3) d'environ 0,002% en poids ou moins, (4) au moins de l'ordre de 100 ppm ou moins, ou (5) de l'ordre de plusieurs centaines de parties par million ou moins.

3. Procédé de neutralisation de la toxicité du nickel dans un bio-alliage de Co-Cr-Mo ou d'un alliage d'acier inoxydable sans Ni selon les revendications 1 ou 2, **caractérisé en ce que** l'on effectue un traitement thermique à une température allant de 600°C à 1250°C avant fusion de l'alliage.

4. Bio-alliage de Co-Cr-Mo ou alliage d'acier inoxydable sans Ni dans lequel Ni est inévitablement présent dans l'alliage et dans lequel la toxicité du nickel est neutralisée par le procédé selon la revendication 1, **caractérisé en ce que** le bio-alliage de Co-Cr-Mo ou l'alliage d'acier inoxydable sans Ni est tel qu'un élément sélectionné parmi Zr ou Ti est ajouté à la composition d'alliage afin de neutraliser la bio-toxicité due à une impureté sous la forme de trace de Ni, les rapports de l'élément additionnel ajouté à la composition d'alliage étant de 0,0001 à 1,0% en poids de Zr ou de 0,1 à 0,5% en poids de Ti pour 1% en poids de Ni.

5. Alliage selon la revendication 4, **caractérisé en ce que** l'alliage a une teneur en nickel (1) d'environ 1,0% en poids ou moins, (2) d'environ 0,5% en poids ou moins, (3) d'environ 0,002% en poids ou moins, (4) au moins de l'ordre de 100 ppm ou moins, ou (5) de l'ordre de plusieurs centaines de parties par million ou moins.

6. Bio-alliage de Co-Cr-Mo ou alliage d'acier inoxydable sans Ni dans lequel la bio-toxicité du nickel est neutralisée conformément aux revendications 4 et 5, **caractérisé en ce que** le traitement thermique à une température allant de 600°C à 1250°C est effectué avant fusion de l'alliage.

7. Dispositif médical fabriqué à partir du bio-alliage de Co-Cr-Mo ou de l'alliage d'acier inoxydable sans Ni dans lequel la toxicité du nickel est neutralisée conformément à la revendication 4.

8. Dispositif médical fabriqué en soumettant le bio-alliage de Co-Cr-Mo ou l'alliage d'acier inoxydable sans Ni, dans lequel la toxicité du nickel est neutralisée conformément à la revendication 4, à un processus sélectionné dans le groupe incluant la trempe, l'atomatisation de métal sous flux gazeux, l'alliage mécanique, la trempe liquide, l'extrusion à chaud, le laminage à chaud, l'étirage à chaud et le forgeage.

Fig. 1

■ Ni  ■ Ni-Ti  □ Ni-Zr  ▦ Ni-Al  ☐ Ni-Nb

Fig. 2

■ Ni  ■ Ni-Ti  □ Ni-Zr  ▦ Ni-Al  ☐ Ni-Nb

Fig. 3

Fig. 4

Fig. 5

Legend: ■ Ni-Ti ☐ Ni-Zr ▦ Ni-Al ☐ Ni-Nb

Y-axis: METAL ELUTION RATE $w/\mu g \cdot cm^{-2}$ (0, 0.05, 0.1, 0.15)

X-axis: Ti, Zr, Al, Nb

Fig. 6

Legend: —◇— Ni  —■— Ni-Zr

Y-axis: NOMINAL STRESS (MPa) (0, 200, 400, 600, 800, 1000, 1200)

X-axis: NOMINAL STRAIN (0, 0.1, 0.2, 0.3, 0.4)

Fig. 7

Co

Fig. 8

Cr

Fig. 9

Fig. 10

Fig. 11

Change in HCP to FCC transformation by 1.0% addition, °C

Fig. 12

(a)

(b)

(c)

Fig. 13

Fig. 14

(a)

100μm

(b)

(c)

(d)

Fig. 15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0047140 A
- JP 2002363675 A
- WO 9700978 A
- US 5462575 A
- US 4668290 A
- JP 49135820 A
- JP 62080245 A
- JP 5001345 A
- US 3591362 A

**Non-patent literature cited in the description**

- **C. T. SIMS ; N. S. STOLOFF ; W. C. HAGEL.** SUPERALLOYS II. Wiley-Interscience, 1987
- **R. C. GEBAU ; R. S. BROWN.** *Adv. Mater. Process.,* 2001, vol. 159, 46-48